# EUROPÄISCHE PATENTSCHRIFT

(11) **EP 2 490 992 B1**
(45) Veröffentlichungstag und Bekanntmachung des Hinweises auf die Patenterteilung: **29.01.2014**
(21) Anmeldenummer: 10765445.1
(22) Anmeldetag: 14.10.2010
(51) Int. Cl.: C07C 29/32, C07C 29/56, C07C 33/20, C07C 45/29, C07C 47/228

(54) **VERFAHREN ZUR HERSTELLUNG M- ODER P-SUBSTITUIERTER PHENYLALKANOLE DURCH ALKYLIERUNG**
PROCESS FOR PREPARING M- OR P-SUBSTITUTED PHENYLALKANOLS BY ALKYLATION
PROCÉDÉ DE FABRICATION D'ALCANOLS PHÉNYLIQUES À SUBSTITUTION M OU P PAR ALKYLATION

(30) Priorität: 23.10.2009 EP 09173910
(43) Veröffentlichungstag der Anmeldung: 29.08.2012
(73) Patentinhaber: BASF SE, 67056 Ludwigshafen (DE)
(72) Erfinder: LANVER, Andreas, 68165 Mannheim (DE); EBEL, Klaus, 68623 Lampertheim (DE); BECK, Karl, 76684 Östringen (DE); PELZER, Ralf, 37699 Fürstenberg (DE); BOTZEM, Jörg, 67117 Limburgerhof (DE); GRIESBACH, Ulrich, 68305 Mannheim (DE)
(86) Internationale Anmeldenummer: PCT/EP2010/065466
(87) Internationale Veröffentlichungsnummer: WO 2011/048012

(56) Entgegenhaltungen:
- EP-A1- 0 045 571
- JP-A- 2 009 830

## Beschreibung

Gegenstand der vorliegenden Erfindung ist ein Verfahren zur Herstellung von m- oder p-alkylsubstituierten Phenylalkanolen durch Friedel-Crafts Alkylierung von Phenylalkanolen und anschliessender Isomerisierung der unerwünschten Nebenprodukte hin zu dem gesuchten m- oder p-alkylsubstituierten Phenylalkanol. Die m- oder p-alkylsubstituierten Phenylalkanole sowie die aus diesen hergestellten m- oder p-alkylsubstituierten Phenylalkanale, beispielsweise Derivate des Geruchstoffs 3-Phenyl-1-propanol, sind als Aromachemikalien von Interesse.

Zur Herstellung alkylsubstituierter Phenylalkanole und deren Derivate sind verschiedene Synthesen bekannt.

In der WO 2008/053148 ist eine 3-stufige Synthese zur Herstellung von 3-(3-tert-Butylphenyl)propanal ausgehend von 1-tert-Butyl-3-ethylbenzol beschrieben. Dabei wird die Ausgangsverbindung zunächst zum 1-tert-Butyl-3-(1-brom-ethyl)benzol bromiert und anschließend zu dem entsprechend substituierten Styrol eliminiert. Durch Hydroformylierung erhält man anschliessend das 3-(3-tert-Butylphenyl)propanal. Für einen grosstechnischen Prozess erscheint diese Synthese aufgrund geringer Ausbeuten weniger geeignet.

Die Herstellung von 2-Methyl-3-(3-tert-butylphenyl)propanal und von 2-Methyl-3-(3-isobutylphenyl)propanal gelingt Ishii et al. (J. Org. Chem. 2005, 70, 5471-5474) durch Palladium-Katalysierte oxidative Kupplung von tert-Butylbenzol bzw. Isopropylbenzol mit Methacrolein gefolgt von einer Palladium-Katalysierten Hydrierung. Bei dem Kupplungsschritt wird ein Katalysatorsystem bestehend aus Pd(OAc)₂ und H₄PMo₁₁VO₄₀ x 26 H₂O eingesetzt. Es wird eine hohe Katalysatormenge von ca. 7 mol% benötig. Bei einer Ausbeute von ca. 65% beträgt das m/p-Verhältnis 56/44 (für 2-Methyl-3-(3-tert-butylphenyl)propanal) bzw. 51/40 (für 2-Methyl-3-(3-iso-butylphenyl)propanal). Auch dieses Verfahren erscheint für einen grosstechnischen Prozess weniger geeignet.

In der EP 0 045 571 ist die Friedel-Crafts Alkylierung von 2-Methyl-3-phenylpropanol zu 2-Methyl-3-(3-tert-butylphenyl)propanol und 2-Methyl-3-(4-tert-butylphenyl)propanol beschrieben. Als Alkylierungsreagenzien werden Isobutylen, Diisobutylen und tert-Butylchlorid eingesetzt. Als Katalysatoren werden Eisenchlorid und Phosphorsäure und als Lösungsmittel Methylenchlorid oder Phosphorsäure verwendet. In Abhängigkeit der Reaktionsbedingungen und des Katalysators werden m/p-Verhältnisse von 1/13 bis 1/5 erhalten. Die Gesamtausbeuten (m-Isomer und p-Isomer) betragen bis zu 52%.

In der DE 29 52 719 wird ebenfalls die Eisenchlorid-Katalysierte Friedel-Crafts Alkylierung von 2-Methyl-3-phenylpropanol beschrieben. In Cyclohexan oder Dichlorethan als Lösungsmittel wurde eine Ausbeute von 84-86% an 2-Methyl-3-(4-tert-butylphenyl)-propanol erhalten. Die Bildung der m-isomeren Verbindung, (2-Methyl-3-(3-tert-butylphenyl)propanol), wurde nicht nachgewiesen. Nachteil der beschrieben Friedel-Crafts Alkylierungen ist die nicht identifizierte Menge der gebildeten m-isomeren Verbindung (Verhältnis m : p ist max. 1 : 5).

Gegenüber diesen bekannten Verfahren ermöglicht das erfindungsgemässe Verfahren, dass man die m-substituierten Phenylalkanole oder die p-substituierten Phenylalkanole, die als Vorstufe für die sehr interessanten entsprechend substituierten Phenylalkanale (Phenylalkylaldehyde) dienen, kostengünstig und in guter Ausbeute und auf einfachste Weise herstellt.

Die vorliegende Erfindung betrifft ein Verfahren zur Herstellung von in m- oder p-Stellung substituierten Phenylalkanolen, welche durch Friedel-Crafts Alkylierung von unsubstituierten Phenylalkanolen zusammen mit Alkylhalogeniden erhalten werden können. Die Alkylierung erfolgt an bestimmten Friedel-Crafts Katalysatoren. Gegenstand der Erfindung ist somit ein Verfahren zur Herstellung m- oder p-alkylsubstituierter Phenylalkanole der Formel (I) 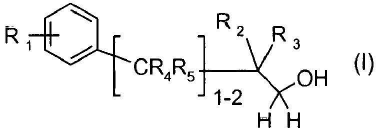 worin R₁ in m- oder p-Stellung an den Phenylring gebunden ist und C₁-C₅-Alkyl bedeutet und R₂, R₃, R₄ und R₅ unabhängig voneinander Wasserstoff oder Methyl bedeuten, welches dadurch gekennzeichnet ist, dass man ein unsubstituiertes Phenylalkanol der Formel (II) 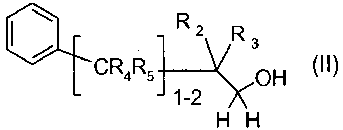 worin R₂, R₃, R₄ und R₅ die unter Formel (I) angegebenen Bedeutungen haben zusammen mit einem C₁-C₅-Alkylhalogenid der Formel (III)

R₁-Hal (III),

worin R₁ die unter Formel (I) angegebene Bedeutung hat und Hal Halogen bedeutet, in Gegenwart eines Friedel-Crafts Katalysators zu einem Gemisch m- und p-alkylsubstituierter Phenylalkanole der Formel (I) alkyliert und anschliessend das Reaktionsgemisch, vorzugsweise in Gegenwart von Wasser bei alkalischem pH-Wert, aufarbeitet und das gewünschte m-alkylsubstituierte Phenylalkanol der Formel (I) oder das gewünschte p-alkylsubstituierte Phenylalkanol der Formel (I) abtrennt, vorzugsweise destillativ abtrennt, und die übrigen gebildeten Nebenprodukte in das Reaktionsgemisch zurückführt und diese in Gegenwart eines Friedel-Crafts Katalysators zu dem gewünschten m- oder p-alkylsubstituierten Phenylalkanol isomerisiert.

Um das m- und p-Verhältnis der Alkylierungsreaktion in die gewünschte Richtung zu beeinflussen, findet nach der Alkylierung und nach der Abtrennung des gewünschten Produktes, also entweder des m-alkylsubstituierten Phenylalkanols oder des p-alkylsubstituierten Phenylalkanols, noch eine Isomerisierung der nicht erwünschten Komponenten zu der gewünschten Komponente statt. Der als Isomerisierung gekennzeichnete Reaktionsschritt bezeichnet die Einstellung eines Gleichgewichts der verschiedenen Komponenten der Umsetzung unter den in Gegenwart eines Friedel-Crafts Katalysators vorliegenden Bedingungen. Im Anschluss an den Isomerisierungsschritt erfolgt eine Aufarbeitung, vorzugsweise wässrige Aufarbeitung, vorzugsweise bei alkalischem pH-Wert, ganz besonders bevorzugt ist eine wässrige Aufarbeitung in Gegenwart von Alkalilauge, wie z.B. Natronlauge und/oder Kalilauge, dann wird das gewünschte Produkt abgetrennt, vorzugsweise destillativ abgetrennt, und die nicht gewünschten Produkte erneut einer Isomerisierung unterzogen. Der Isomerisierungsschritt kann quasi kontinuierlich erfolgen, indem nach jedem Isomerisierungsschritt eine wässrige Aufarbeitung erfolgt, vorzugsweise unter alkalischen Bedingungen wie oben angegeben, mit nachfolgender Abtrennung, vorzugsweise destillativer Abtrennung, um die Ausbeute des gewünschten Produktes zu erhöhen.

Die wässrige Aufarbeitung der Reaktionsmasse gemäss dem vorliegenden Verfahren erfolgt nach der Alkylierung und auch nach dem Isomerisierungsschritt indem die Reaktionsmasse bei Raumtemperatur mit Wasser, vorzugsweise bei alkalischem pH-Wert, besonders bevorzugt in Gegenwart von Alkalilauge, insbesondere Natronlauge oder Kalilauge, versetzt wird. Nachfolgend wird das gewünschte Reaktionsprodukt abgetrennt, vorzugsweise durch Destillation.

Es wurde gefunden, dass bei der Alkylierung von unsubstituierten Phenylalkanolen zu alkylsubstituierten Phenylalkanolen unter bestimmten Bedingungen m/p-Isomerenverhältnisse von > 1,5/1 erhalten werden können. Reine m- oder p-substituierte Phenylalkanole der Formel (I) sind auf diesem Weg kaum zu erhalten, so dass an die Alkylierung eine Isomerisierung der unerwünschten Nebenprodukte zu einem der gewünschten Reaktionsprodukte angeschlossen wurde.

Es ist natürlich ebenfalls möglich die m-alkylsubstituierte Komponente und die p-alkylsubstituierte Komponente der Formel (I) z.B. durch fraktionierte Destillation abzutrennen und die übrigen Nebenprodukte erneut der Reaktionsmasse zuzuführen, um durch Isomerisierung die Ausbeute beider Komponenten zu erhöhen.

Als C₁-C₅-Alkylhalogenide kommen in dem erfindungsgemässen Verfahren z.B. in Betracht: Methylbromid, Methyljodid, Ethylbromid, Ethyljodid, n-Propylchlorid, n-Propylbromid, n-Propyljodid, Isopropylchlorid, Isopropylbromid, Isopropyljodid, n-Butylchlorid, n-Butylbromid, Isobutylchlorid, Isobutylbromid, sec-Butylchlorid, sec-Butylbromid, tert-Butylchlorid, tert-Butylbromid, n-Pentylchlorid, n-Pentylbromid.

Bevorzugte C₁-C₅-Alkylhalogenide der Formel (III) sind Ethylhalogenid, insbesondere Ethylbromid und Ethyljodid, Isopropylhalogenid, insbesondere Isopropylchlorid und Isopropylbromid, Isobutylhalogenid, insbesondere Isobutylchlorid und Isobutylbromid, tert-Butylhalogenid, insbesondere tert-Butylchlorid.

Bevorzugt ist ein Verfahren zur Herstellung m- oder p-substituierter Phenylpropanole der Formel (IV) 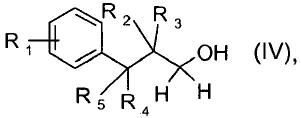 worin R₁, R₂, R₃, R₄ und R₅ die unter Formel (I) angegebenen Bedeutungen haben, das dadurch gekennzeichnet ist, dass man ein unsubstituiertes Phenylpropanol der Formel (V) 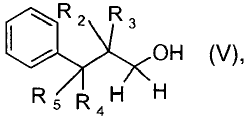 worin R₂, R₃, R₄ und R₅ die unter Formel (IV) angegebenen Bedeutungen haben zusammen mit einem C₁-C₅-Alkylhalogenid der Formel (III)

R₁-Hal (III),

worin R₁ und Hal die unter Formel (III) angegebenen Bedeutungen haben, in Gegenwart eines Friedel-Crafts Katalysators zu einem Gemisch m- und p-alkylsubstituierter Phenylpropanol der Formel (IV) alkyliert, anschliessend das Reaktionsgemisch, vorzugsweise in Gegenwart von Wasser bei alkalischem pH-Wert, aufarbeitet und das gewünschte m- oder p-alkylsubstituierte Phenylpropanol der Formel (IV) abtrennt, vorzugsweise destillativ abtrennt, die übrigen gebildeten Nebenprodukte in das Reaktionsgemisch zurückführt und diese in Gegenwart eines Friedel-Crafts Katalysators zu dem gewünschten m- oder p-alkylsubstituierten Phenylpropanol isomerisiert.

Besonders bevorzugt ist ein Verfahren welches dadurch gekennzeichnet ist, dass man von einem unsubstituierten Phenylpropanol der Formel (VI) ausgeht 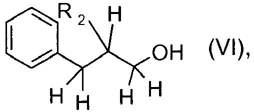 worin R₂ die unter Formel (I) angegebene Bedeutung hat, dieses zusammen mit einem C₁-C₅-Alkylhalogenid der Formel (III)

R₁-Hal (III),

worin R₁ und Hal die unter Formel (III) angegebenen Bedeutungen haben, in Gegenwart eines Friedel-Crafts-Katalysators zu einem m- oder p-alkylsubstituierten Phenylpropanol der Formel (VII) 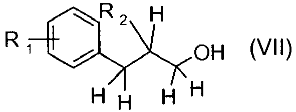 umsetzt, worin R₁ und R₂ die unter Formel (I) angegebene Bedeutung haben, anschliessend das Reaktionsgemisch, vorzugsweise in Gegenwart von Wasser bei alkalischem pH-Wert, aufarbeitet und das gewünschte m- oder p-alkylsubstituierte Phenylpropanol der Formel (VII) abtrennt, vorzugsweise destillativ abtrennt, die übrigen gebildeten Nebenprodukte in das Reaktionsgemisch zurückführt und diese in Gegenwart eines Friedel-Crafts Katalysators zu dem gewünschten m- oder p-substituierten Phenylpropanol isomerisiert.

Ein ganz besonders bevorzugtes Verfahren ist dadurch gekennzeichnet, dass man als Ausgangsverbindung 2-Methyl-3-phenylpropanol zusammen mit tert-Butylhalogenid, insbesondere tert-Butylchlorid, in Gegenwart von Aluminiumtrichlorid (AlCl₃) zu 2-Methyl-3-(3- oder 4-tert-butylphenyl)propanol umsetzt, anschliessend das Reaktionsgemisch, vorzugsweise in Gegenwart von Wasser bei alkalischem pH-Wert, aufarbeitet und das gewünschte 3-Methyl-3-(3- oder 4-tert-butylphenyl)-propanol abtrennt, vorzugsweise destillativ abtrennt, und die übrigen gebildeten Nebenprodukte in das Reaktionsgemisch zurückführt und diese in Gegenwart des Friedel-Crafts Katalysators zu dem gewünschten 3-Methyl-3-(3- oder 4-tert-butylphenyl)-propanol isomerisiert.

Ein ebenfalls besonders bevorzugtes Verfahren ist dadurch gekennzeichnet, dass man als Ausgangverbindung 2-Methyl-3-phenylpropanol, 3-Phenylpropanol, 3-Phenyl-2,2-dimethylpropanol oder 3-Phenylbutanol einsetzt und die Alkylierung in Gegenwart eines Isobutylhalogenids, vorzugsweise Isobutylchlorids, tert-Butylhalogenids, vorzugsweise tert-Butylchlorids, lsopropylhalogenids, vorzugsweise Isopropylchlorids, Ethylhalogenids, vorzugsweise Ethylbromids, und in Gegenwart eines Friedel-Crafts-Katalysators zu den Verbindungen 2-Methyl-3-(3- oder 4-iso-butylphenyl)-propanol, 3-(3- oder 4-tert-Butylphenyl)propanol, 2-Methyl-3-(3- oder 4-isopropylphenyl)propanol, 3-(3-oder 4-Ethylphenyl)-2,2-dimethylpropanol oder 3-(3- oder 4-Isopropylphenyl)-butanol durchführt und anschliessend das Reaktionsgemisch, vorzugsweise in Gegenwart von Wasser bei alkalischem pH-Wert, aufarbeitet und das gewünschte Produkt abtrennt, vorzugsweise destillativ abtrennt, und die übrigen gebildeten Nebenprodukte in das Reaktionsgemisch zurückführt und in Gegenwart eines Friedel-Crafts Katalysators zu dem gewünschten Produkt isomerisiert.

Eine weitere bevorzugte Ausführungsform ist dadurch gekennzeichnet, dass man nach Aufarbeitung des Reaktionsgemisches, vorzugsweise wässriger Aufarbeitung bei alkalischem pH-Wert, das gewünschte m-substituierte Reaktionsprodukt abtrennt, d.h. dass man vorzugsweise das m-alkylsubstituierte Phenylalkanol der Formel (I), bzw. das malkylsubstituierte Phenylpropanol der Formel (IV), bzw. das m-alkylsubstituierte Phenylpropanol der Formel (VII), bzw. das 3-Methyl-3-(3-tert-butylphenyl)-propanol, abtrennt bzw. die Verbindungen 2-Methyl-3-(3-iso-butylphenyl)-propanol, 3-(3-tert-Butylphenyl)propanol, 2-Methyl-3-(3-isopropylphenyl)propanol, 3-(3-Ethylphenyl)-2,2-dimethylpropanol oder 3-(3-Isopropylphenyl)butanol abtrennt.

Als Katalysatoren können typische Friedel-Crafts Katalysatoren eingesetzt werden. Als Beispiele seien genannt AlCl₃, AlBr₃, TiCl₄, ZrCl₄, VCl₃, ZnCl₂, FeBr₃ und FeCl₃. Bevorzugt werden die Friedel-Crafts Katalysatoren AlCl₃ oder AlBr₃ verwendet. Es werden im allgemeinen Katalysatormengen von 1 bis 200 mol-% bezogen auf die Molmenge der eingesetzten Phenylalkanolverbindung eingesetzt. Bevorzugt sind Katalysatormengen von 33% bis 110 mol-% bezogen auf die Molmenge der eingesetzten Phenylalkanolverbindung.

Die Alkylierung und auch die Isomerisierung erfolgen bei Temperaturen zwischen 0° C und 100°C. Besonders bevorzugt sind Temperaturen zwischen 10°C und 50°C. Die Reaktionszeiten betragen 30 Minuten bis 24 Stunden. Besonders bevorzugt sind Reaktionszeiten zwischen 1 Stunde und 6 Stunden.

Die Alkylierungsreaktion sowie auch die Isomerisierungsreaktion kann lösungsmittelfrei oder in einem Lösungsmittel durchgeführt werden. Geeignete Lösungsmittel sind: Cyclohexan, Toluol, p-tert-Butyltoluol, Dichlormethan, 1,2-Dichlorethan, Chlorbenzol, Dichlorbenzol. Besonders bevorzugt sind Dichlormethan und Chlorbenzol.

Bevorzugte Ausgangsstoffe für die Alkylierung sind die folgenden Substrate: 2-Methyl-3-phenylpropanol, 2-Methyl-3-phenylpropanol, 3-Phenylpropanol, 2-Methyl-3-phenylpropanol, 3-Phenyl-2,2-dimethylpropanol, 3-Phenylbutanol. Bevorzugte Alkylierungsmittel sind: Isobutylchlorid, tert-Butylchlorid, Isopropylchlorid, Ethylbromid. Daraus ergeben sich die folgenden m-Isomere als Hauptprodukte der Reaktion: 2-Methyl-3-(3-tert-butylphenyl)propanol, 2-Methyl-3-(3-iso-butylphenyl)propanol, 3-(3-tert-Butylphenyl)propanol, 2-Methyl-3-(3-isopropylphenyl)propanol, 3-(3-Ethylphenyl)-2,2-dimethylpropanol, 3-(3-Isopropylphenyl)butanol. Aus der Reaktion ergeben sich ferner die folgenden p-Isomere als Produkte der Reaktion: 2-Methyl-3-(4-tert-butylphenyl)propanol, 2-Methyl-3-(4-iso-butylphenyl)propanol, 3-(4-tert-Butylphenyl)propanol, 2-Methyl-3-(4-isopropylphenyl)propanol, 3-(4-Ethylphenyl)-2,2-dimethylpropanol, 3-(4-Isopropylphenyl)butanol. Besonders bevorzugt sind die Substrate 2-Methyl-3-(3- oder 4-tert-butylphenyl)propanol.

Die Reaktion wird in der Regel so durchgeführt, dass der Katalysator zusammen mit dem Alkylhalogenid der Formel (III) in das im Lösungsmittel gelöste Phenylalkanol eingetragen wird. Die Aufarbeitung erfolgt durch Aufarbeitung mit Wasser und ggf. Natronlauge, sowie durch Destillation des Lösungsmittels. Die Aufreinigung des Rohproduktes und die Isolierung des gewünschten m- oder p-substituierten Phenylalkanols erfolgt in der Regel durch Destillation.

Die bei der Reaktion entstehenden am Aromaten mono- und tri-substituierten Phenylalkanole sowie das gegebenenfalls unerwünschte m- oder p-alkylsubstituierte Phenylalkanol der Formeln (A), (B) und (C) 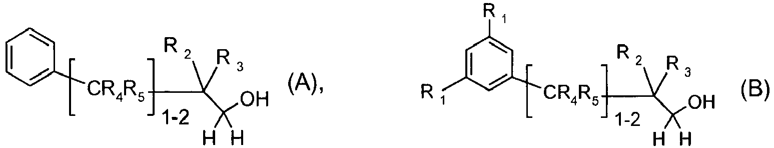 und 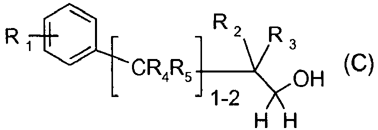 worin R₁, R₂, R₃, R₄ und R₅ die unter Formel (I) angegebenen Bedeutungen haben, werden der Reaktionsmasse nach vorzugsweise wässriger Aufarbeitung, vorzugsweise bei alkalischem pH-Wert, und vorzugsweise destillativer Abtrennung wieder zugeführt. Durch die Rückführung dieser Produkte in die Reaktionsmasse wird das Gleichgewicht zwischen der p-substituierten und der m-substituierten Komponente und den mono- und tri-substituierten Phenylalkanolen immer wieder neu eingestellt, wodurch ein erhöhter Anteil an dem gewünschten m- oder p-substituierten Produkt erhalten wird, da dieses gewünschte Produkt der Reaktionsmasse vor jeder Rückführung (nach Aufarbeitung und Destillation) entzogen wird. Vorteilhafterweise erfolgt die Umsetzung als eine Eintopf-Reaktion, worin in einem 1. Schritt die Alkylierung durchgeführt wird, die Reaktionsprodukte zusammen mit unumgesetztem Ausgangsmaterial nach wässriger Aufarbeitung vorzugsweise destillativ abgetrennt werden, die Fraktion des gewünschten m- oder p- substituierten Phenylalkanols aufgefangen wird und die Nebenprodukte der Formeln (A), (B) und (C) in das Reaktionsgefäss zurückgeführt werden und in einem 2. Schritt erneut in Gegenwart eines Friedel-Crafts Katalysators gegebenenfalls in einem Lösungsmittel unter den angegebenen Bedingungen zu der gewünschten m- oder p-substituierten Phenylalkanolverbindung isomerisiert werden. Die Isomerisierung mit nachfolgender wässriger Aufarbeitung und destillativer Abtrennung der gewünschten Komponente sowie der Rückführung der übrigen Komponenten der Formeln (A), (B) und (C) zur erneuten Isomerisierung kann mehrfach bzw. quasi kontinuierlich erfolgen, um den Anteil der gewünschten Komponente, m- oder p-alkylsubstituiertes Phenylalkanol, in hoher Ausbeute zu erhalten.

Die erfindungsgemäss hergestellten Alkanole der Formel (I) können, basierend auf an sich bekannten Dehydrierungs- oder Oxidationsmethoden, in die entsprechenden Aldehyde überführt werden (vergl. z.B.: Houben-Weyl" Methoden der organischen Chemie" , Band 7/1, S. 160ff, S. 171f). Besonders interessante Verbindungen dieser Substanzklasse sind 2-Methyl-3-(3- oder 4-tert-butylphenyl)propanal, 2-Methyl-3-(3- oder 4-iso-butylphenyl)propanal, 3-(3- oder 4-tert-Butylphenyl)propanal, 2-Methyl-3-(3- oder 4-isopropylphenyl)-propanal, 3-(3- oder 4-Ethylphenyl)-2,2-dimethylpropanal und 3-(3- oder 4-Isopropylphenyl)butanal.

Wie in der EP-A-0 045 571 beschrieben können Phenylpropanole durch Oxidation oder Dehydrierung in die entsprechenden Phenylpropanale überführt werden. Diese Umsetzung gelingt beispielsweise durch Kupferchromit-katalysierte Flüssigphasendehydrierung.

Bevorzugte Edukte zur Umsetzung zum Aldehyd sind 2-Methyl-3-(3- oder 4-tert-butylphenyl)-propanol, 2-Methyl-3-(3- oder 4-iso-butylphenyl)propanol, 3-(3- oder 4-tert-Butylphenyl)propanol, 2-Methyl-3-(3- oder 4-isopropylphenyl)propanol, 3-(3- oder 4-Ethylphenyl)-2,2-dimethylpropanol, 3-(3- oder 4-Isopropylphenyl)butanol. Daraus ergeben sich durch Oxidation bzw. Dehydrierung die folgenden Aldehyde: 2-Methyl-3-(3- oder 4-tert-butylphenyl)propanal, 2-Methyl-3-(3- oder 4-iso-butylphenyl)propanal, 3-(3- oder 4-tert-Butylphenyl)propanal, 2-Methyl-3-(3- oder 4-isopropylphenyl)-propanal, 3-(3- oder 4-Ethylphenyl)-2,2-dimethylpropanal, 3-(3- oder 4-Isopropylphenyl)butanal.

Ein weiterer Gegenstand der Erfindung ist somit die Herstellung der aus den m- oder p-substituierten Phenylalkanolen der Formel (I) durch Oxidation oder Dehydrierung erhältlichen Wertprodukte der Formel (VIII), die als Duftstoffe und Aromachemikalien eine interessante Rolle spielen, 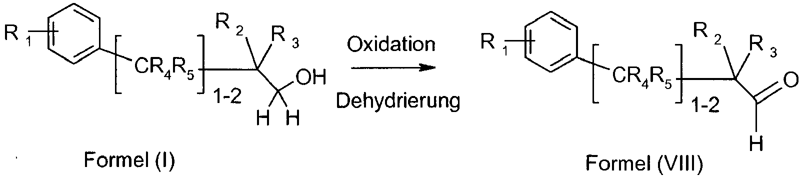 worin R₁, R₂, R₃, R₄ und R₅ die unter Formel (I) angegebenen Bedeutungen haben. Das erfindungsgemässe Verfahren zur Herstellung von Duft- und Aromastoffen der Formel (VIII) 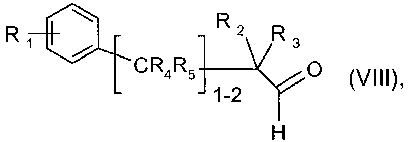 worin R₁ in m- oder p-Stellung an den Phenylring gebunden ist und C₁-C₅-Alkyl bedeutet und R₂, R₃, R₄ und R₅ unabhängig voneinander Wasserstoff oder Methyl bedeuten, ist dadurch gekennzeichnet, dass man ein unsubstituiertes Phenylalkanol der Formel (II) 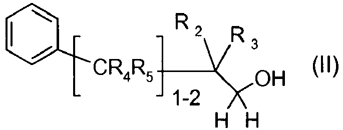 worin R₂, R₃, R₄ und R₅ die unter Formel (VIII) angegebenen Bedeutungen haben zusammen mit einem C₁-C₅-Alkylhalogenid der Formel (III)

R₁-Hal (III),

worin R₁ und Hal die unter Formel (III) angegebenen Bedeutungen haben, in Gegenwart eines Friedel-Crafts Katalysators zu einem Gemisch m- und p-alkylsubtituierter Phenylalkanol der Formel (I) 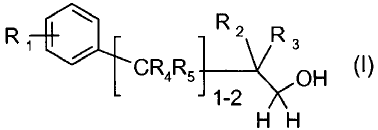 alkyliert, worin R₁, R₂, R₃, R₄ und R₅ die unter Formel (I) angegebenen Bedeutungen haben, anschliessend das Reaktionsgemisch, vorzugsweise in Gegenwart von Wasser bei alkalischem pH-Wert, aufarbeitet, und das gewünschte m- oder p-alkylsubstituierte Phenylalkanol der Formel (I) abtrennt, vorzugsweise destillativ abtrennt, und die übrigen gebildeten Nebenprodukte in das Reaktionsgemisch zurückführt und diese in Gegenwart eines Friedel-Crafts Katalysators zu dem gewünschten m- oder p-alkylsubstituierten Phenylalkanol isomerisiert, und anschliessend das erhaltene m-oder p-alkylsubstituierte Phenylalkanol der Formel (I) durch Oxidation oder Dehydrierung in das m- oder p-alkylsubstituierte Phenylalkanal der Formel (VIII) überführt.

### Die Erfindung wird durch die folgenden Beispiele näher veranschaulicht. In den Beispielen verstehen sich alle Angaben in % als mol-%.

### Beispiele

### Beispiel 1

15,1 g (100 mmol) 2-Methyl-3-phenylpropanol wurden in 87g Dichlormethan vorgelegt. Innerhalb von 4 Stunden wurden 13,4g (100 mmol) AlCl₃ und 9,3g (100mmol) tert-Butylchlorid bei einer Temperatur von 1-10°C zugegeben. Es wurde auf Raumtemperatur erwärmt, mit Wasser und Natronlauge aufgearbeitet und das Lösungsmittel entfernt. Es wurde ein Gemisch mit der folgenden Zusammensetzung erhalten: 2-Methyl-3-(3-tert-butylphenyl)propanol (41%); 2-Methyl-3-(4-tert-butylphenyl)propanol (24%); 3-(3,5-Di-tert-butylphenyl)-2-methyl-propanol (19%); 2-Methyl-3-phenylpropanol (15%).

### Beispiel 2

15,1 g (100 mmol) 2-Methyl-3-phenylpropanol wurden in 78g Dichlormethan vorgelegt. Innerhalb von 1,5 Stunden wurden 16,2g (100 mmol) FeCl₃ und 9,3g (100mmol) tert-Butylchlorid bei einer Temperatur von 1-5°C zugegeben. Es wurde auf Raumtemperatur erwärmt, mit Wasser und Natronlauge aufgearbeitet und das Lösungsmittel entfernt. Es wurde ein Gemisch mit der folgenden Zusammensetzung erhalten: 2-Methyl-3-(3-tert-butylphenyl)propanol (36%); 2-Methyl-3-(4-tert-butylphenyl)propanol (24%); 3-(3,5-Di-tert-butylphenyl)-2-methyl-propanol (27%); 2-Methyl-3-phenylpropanol (7,5%).

## Patentansprüche

1. Verfahren zur Herstellung m- oder p-substituierter Phenylalkanole der Formel (I) 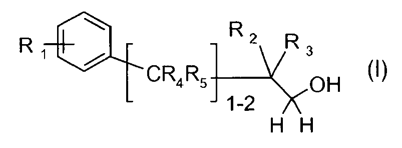 worin R₁ in m- oder p-Stellung an den Phenylring gebunden ist und C₁-C₅-Alkyl bedeutet, und R₂, R₃, R₄ und R₅ unabhängig voneinander Wasserstoff oder Methyl bedeuten, **dadurch gekennzeichnet, dass** man ein unsubstituiertes Phenylalkanol der Formel (II) 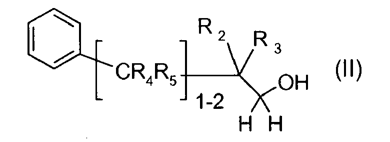 worin R₂, R₃, R₄ und R₅ die unter Formel (I) angegebenen Bedeutungen haben zusammen mit einem C₁-C₅-Alkylhalogenid der Formel (III)
R₁-Hal (III),
worin R₁ die unter Formel (I) angegebene Bedeutung hat und Hal Halogen bedeutet, in Gegenwart eines Friedel-Crafts Katalysators zu einem Gemisch m- und p-alkylsubstituierter Phenylalkanole der Formel (I) alkyliert, anschliessend das Reaktionsgemisch aufarbeitet, und das gewünschte m-alkylsubstituierte Phenylalkanol der Formel (I) oder das gewünschte p-alkylsubstituierte Phenylalkanol der Formel (I) abtrennt, die übrigen gebildeten Produkte in das Reaktionsgemisch zurückführt und diese in Gegenwart eines Friedel-Crafts Katalysators zu dem gewünschten m- oder p-alkylsubstituierten Phenylalkanol isomerisiert.

2. Verfahren gemäss Anspruch 1 zur Herstellung m- oder p-substituierter Phenylpropanole der Formel (IV) 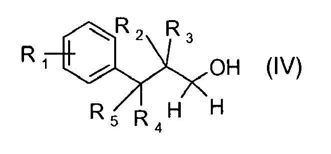 worin R₁, R₂, R₃, R₄ und R₅ die in Anspruch 1 angegebenen Bedeutungen haben, **dadurch gekennzeichnet, dass** man ein unsubstituiertes Phenylpropanol der Formel (V) 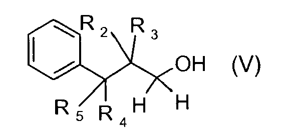 worin R₂, R₃, R₄ und R₅ die unter Formel (IV) angegebenen Bedeutungen haben zusammen mit einem C₁-C₅-Alkylhalogenid der Formel (III)
R₁-Hal (III)
worin R₁ und Hal die in Anspruch 1 angegebenen Bedeutungen haben, in Gegenwart eines Friedel-Crafts Katalysators zu einem Gemisch m- und p-alkylsubstituierter Phenylpropanole der Formel (IV) alkyliert und anschliessend das Reaktionsgemisch aufarbeitet und das gewünschte m- oder p-alkylsubstituierte Phenylpropanol der Formel (IV) abtrennt, vorzugsweise destillativ abtrennt, und die übrigen gebildeten Nebenprodukte in das Reaktionsgemisch zurückführt und diese in Gegenwart eines Friedel-Crafts Katalysators zu dem gewünschten m- oder p-alkylsubstituierten Phenylalkanol isomerisiert.

3. Verfahren gemäss einem der Ansprüche 1 und 2, **dadurch gekennzeichnet, dass** man von einem unsubstituierten Phenylpropanol der Formel (VI) ausgeht 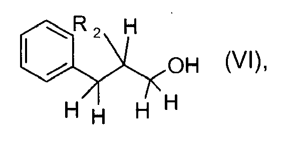 worin R₂ die in Anspruch 1 angegebene Bedeutung hat, dieses zusammen mit einem C₁-C₅-Alkylhalogenid der Formel (III)
R₁-Hal (III),
worin R₁ und Hal die in Anspruch 1 angegebenen Bedeutungen haben, in Gegenwart eines Friedel-Crafts-Katalysators zu einem m- oder p-alkylsubstituierten Phenylpropanol der Formel (VII) 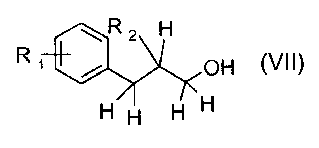 umsetzt, worin R₁ und R₂ die unter Formeln (VI) und (III) angegebenen Bedeutungen haben und anschliessend das Reaktionsgemisch aufarbeitet und das gewünschte m- oder p-alkylsubstituierte Phenylpropanol der Formel (VII) abtrennt, vorzugsweise destillativ abtrennt, und die übrigen gebildeten Nebenprodukte in das Reaktionsgemisch zurückführt und diese in Gegenwart eines Friedel-Crafts Katalysators zu dem gewünschten m- oder p-alkylsubstituierten Phenylpropanol isomerisiert.

4. Verfahren gemäss einem der Ansprüche 1 bis 3, **dadurch gekennzeichnet, dass** man als C₁-C₅-Alkylhalogenid Ethylhalogenid, insbesondere Ethylbromid und Ethyljodid, Isopropylhalogenid, insbesondere Isopropylchlorid und Isopropylbromid, Isobutylhalogenid, insbesondere Isobutylchlorid und Isobutylbromid, oder tert-Butylhalogenid, insbesondere tert-Butylchlorid einsetzt.

5. Verfahren gemäss einem der Ansprüche 1 bis 4, **dadurch gekennzeichnet, dass** man als Friedel-Crafts Katalysator AlCl₃, AlBr₃, TiCl₄, ZrCl₄, VCl₃, ZnCl₂, FeBr₃, FeCl₃ verwendet.

6. Verfahren gemäss Anspruch 5, **dadurch gekennzeichnet, dass** man als Friedel-Crafts Katalysator AlCl₃ oder AlBr₃ verwendet.

7. Verfahren gemäss einem der Ansprüche 1 bis 6, **dadurch gekennzeichnet, dass** man den Friedel-Crafts Katalysator in einer Menge von 1 bis 200 mol-%, insbesondere zwischen 33 und 110 mol-%, bezogen auf die Molmenge des eingesetzten Phenylalkanols, einsetzt.

8. Verfahren gemäss einem der Ansprüche 1 bis 6, **dadurch gekennzeichnet, dass** man die Alkylierung bei einer Temperatur zwischen 0 und 100°C durchführt.

9. Verfahren gemäss einem der Ansprüche 1 bis 8, **dadurch gekennzeichnet, dass** man 2-Methyl-3-phenylpropanol zusammen mit tert-Butylhalogenid, insbesondere tert-Butylchlorid in Gegenwart eines Friedel-Crafts Katalysators, insbesondere in Gegenwart von Aluminiumtrichlorid oder Aluminiumtribromid zu 2-Methyl-3-(3- oder 4-tert-butylphenyl)propanol umsetzt, und anschliessend das Reaktionsgemisch aufarbeitet und das gewünschte 2-Methyl-3-(3- oder 4-tert-butylphenyl)propanol abtrennt, vorzugsweise destillativ abtrennt, und die übrigen gebildeten Nebenprodukte in das Reaktionsgemisch zurückführt und diese in Gegenwart eines Friedel-Crafts Katalysators zu dem gewünschten 2-Methyl-3-(3- oder 4-tert-butylphenyl)-propanol isomerisiert.

10. Verfahren gemäss einem der Ansprüche 1 bis 9, **dadurch gekennzeichnet, dass** man nach Aufarbeitung des Reaktionsgemisches das gewünschte m-substituierte Reaktionsprodukt abtrennt.

11. Verfahren zur Herstellung von Duftstoffen der Formel (VIII) 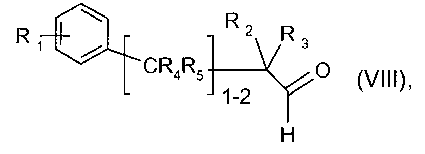 worin R₁ in m- oder p-Stellung an den Phenylring gebunden ist und C₁-C₅-Alkyl bedeutet und R₂, R₃, R₄ und R₅ unabhängig voneinander Wasserstoff oder Methyl bedeuten, **dadurch gekennzeichnet, dass** man ein unsubstituiertes Phenylalkanol der Formel (II) 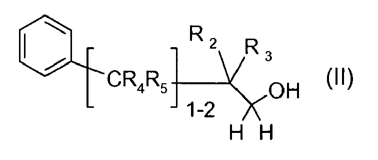 worin R₂, R₃, R₄ und R₅ die unter Formel (VIII) angegebenen Bedeutungen haben zusammen mit einem C₁-C₅-Alkylhalogenid der Formel (III)
R₁-Hal (III),
worin R₁ die unter Formel (VIII) angegebene Bedeutung hat und Hal Halogen bedeutet, in Gegenwart eines Friedel-Crafts Katalysators zu einem m- oder p-alkylsubstituierten Phenylalkanol der Formel (I) 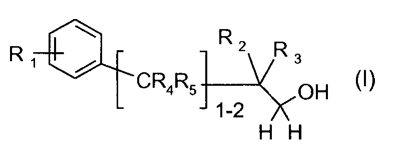 alkyliert, worin R₁, R₂, R₃, R₄ und R₅ die unter Formel (VIII) angegebenen Bedeutungen haben, und anschliessend das Reaktionsgemisch aufarbeitet und das erhaltene m- oder p-alkylsubstituierte Phenylalkanol der Formel (I) abtrennt, vorzugsweise destillativ abtrennt, und die übrigen gebildeten Nebenprodukte in das Reaktionsgemisch zurückführt und diese in Gegenwart eines Friedel-Crafts Katalysators zu dem gewünschten m- oder p-alkylsubstituierten Phenylalkanol isomerisiert, und anschliessend durch Oxidation oder Dehydrierung in das m- oder p-alkylsubstituierte Phenylalkanal der Formel (VIII) überführt.

## Claims

1. A process for the preparation of m- or p-substituted phenylalkanols of the formula (I) 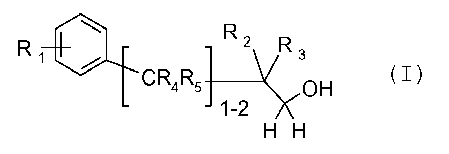 in which R₁ is bonded to the phenyl ring in the m- or p-position and is C₁-C₅-alkyl, and R₂, R₃, R₄ and R₅, independently of one another, are hydrogen or methyl, wherein an unsubstituted phenyl alkanol of the formula (II) 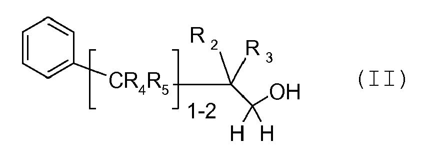 in which R₂, R₃, R₄ and R₅ have the meanings given below formula (I) is alkylated together with a C₁-C₅-alkyl halide of the formula (III)
R₁-Hal (III),
in which R₁ has the meaning given under formula (I) and Hal is halogen, in the presence of a Friedel-Crafts catalyst to give a mixture of m- and p-alkyl-substituted phenylalkanols of the formula (I), then the reaction mixture is worked-up, and the desired m-alkyl-substituted phenylalkanol of the formula (I) or the desired p-alkyl-substituted phenylalkanol of the formula (I) is separated off, the other formed products are returned to the reaction mixture and these are isomerized in the presence of a Friedel-Crafts catalyst to give the desired m- or p-alkyl-substituted phenylalkanol.

2. The process according to claim 1 for the preparation of m- or p-substituted phenylpropanols of the formula (IV) 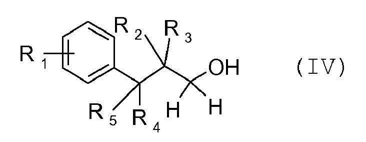 in which R₁, R₂, R₃, R₄ and R₅ have the meanings given in claim 1, wherein an unsubstituted phenylpropanol of the formula (V) 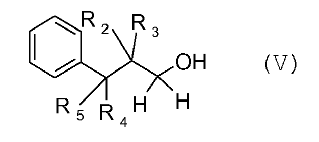 in which R₂, R₃, R₄ and R₅ have the meanings given under formula (IV) is alkylated together with a C₁-C₅-alkyl halide of the formula (III)
R₁-Hal (III)
in which R₁ and Hal have the meanings given in claim 1, in the presence of a Friedel-Crafts catalyst to give a mixture of m- and p-alkyl-substituted phenylpropanols of the formula (IV), and then the reaction mixture is worked-up and the desired m- or p-alkyl-substituted phenylpropanol of the formula (IV) is separated off, preferably separated off by distillation, and the other formed by-products are returned to the reaction mixture and these are isomerized in the presence of a Friedel-Crafts catalyst to give the desired m- or p-alkyl-substituted phenylalkanol.

3. The process according to either of claims 1 and 2, wherein the starting compound used is an unsubstituted phenylpropanol of the formula (VI) 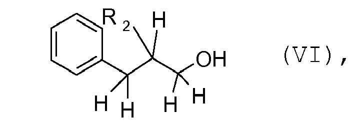 in which R₂ has the meaning given in claim 1, this is reacted together with a C₁-C₅-alkyl halide of the formula (III)
R₁-Hal (III),
in which R₁ and Hal have the meanings given in claim 1, in the presence of a Friedel-Crafts catalyst to give an m- or p-alkyl-substituted phenylpropanol of the formula (VII) 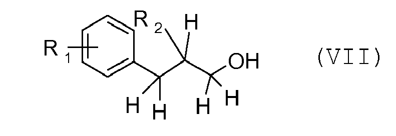 in which R₁ and R₂ have the meanings given under formulae (VI) and (III), and then the reaction mixture is worked-up and the desired m- or p-alkyl-substituted phenylpropanol of the formula (VII) is separated off, preferably separated off by distillation, and the other formed by-products are returned to the reaction mixture and these are isomerized in the presence of a Friedel-Crafts catalyst to give the desierd m- or p-alkyl-substituted phenylpropanol.

4. The process according to any one of claims 1 to 3, wherein the C₁-C₅-alkyl halide used is ethyl halide, in particlar ethyl bromide and ethyl iodide, isopropyl halide, in particular isopropyl chloride and isopropyl bromide, isobutyl halide, in particular isobutyl chloride and isobutyl bromide, or tert-butyl halide, in particular tert-butyl chloride.

5. The process according to any one of claims 1 to 4, wherein the Friedel-Crafts catalyst used is AlCl₃, AlBr₃, TiCl₄, ZrCl₄, VCl₃, ZnCl₂, FeBr₃, FeCl₃.

6. The process according to claim 5, wherein the Friedel-Crafts catalyst used is AlCl3 or AlBr3.

7. The process according to any one of claims 1 to 6, wherein the Friedel-Crafts catalyst is used in an amount of from 1 to 200 mol%, in particular between 33 and 110 mol%, based on the molar amount of the phenylalkanol used.

8. The process according to any one of claims 1 to 6, wherein the alkylation is carried out at a temperature between 0 and 100°C.

9. The process according to any one of claims 1 to 8, wherein 2-methyl-3-phenylpropanol is reacted together with tert-butyl halide, in particular tert-butyl chloride, in the presence of a Friedel-Crafts catalyst, in particular in the presence of aluminum trichloride or aluminum tribromide, to give 2-methyl-3-(3- or 4-tert-butylphenyl)propanol, and then the reaction mixture is worked-up and the desired 2-methyl-3-(3- or 4-tert-butylphenyl)propanol is separated off, preferably separated off by distillation, and the other formed by-products are returned to the reaction mixture and these are isomerized in the presence of a Friedel-Crafts catalyst to give the desired 2-methyl-3-(3- or 4-tert-butylphenyl)propanol.

10. The process according to any one of claims 1 to 9, wherein, following work-up of the reaction mixture, the desired m-substituted reaction product is separated off.

11. A process for the preparation of fragrances of the formula (VIII) 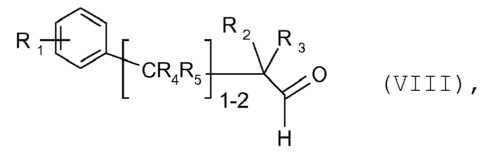 in which R₁ is bonded to the phenyl ring in the m- or p-position and is C₁-C₅-alkyl, and R₂, R₃, R₄ and R₅, independently of one another, are hydrogen or methyl, wherein an unsubstituted phenylalkanol of the formula (II) 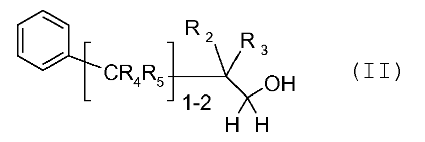 in which R₂, R₃, R₄ and R₅ have the meanings given under formula (VIII) is alkylated together with a C₁-C₅-alkyl halide of the formula (III)
R₁-Hal (III),
in which R₁ has the meaning given under formula (VIII) and Hal is halogen, in the presence of a Friedel-Crafts catalyst to give an m- or p-alkyl-substituted phenylalkanol of the formula (I) 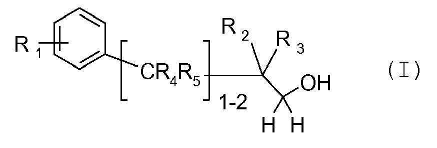 in which R₁, R₂, R₃, R₄ and R₅ have the meanings given under formula (VIII), and then the reaction mixture is worked-up and the resulting m- of p-alkyl-substituted phenylalkanol of the formula (I) is separated off, preferably separated off by distillation, and the other formed by-products are returned to the reaction mixture and these are isomerized in the presence of a Friedel-Crafts catalyst to give the desired m- or p-alkyl-substituted phenylalkanol, and then converted to the m- or p-alkyl-substituted phenylalkanal of the formula (VIII) by oxidation or dehydrogenation

## Revendications

1. Procédé pour la préparation de phénylalcanols m- ou p-substitués de formule (I) 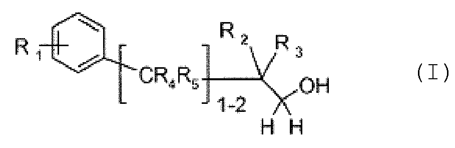 dans laquelle R₁ est lié en position m ou p au cycle phényle et représente un groupe alkyle en C₁-C₅, et R₂, R₃, R₄ et R₅ représentent, indépendamment les uns des autres, un atome d'hydrogène ou le groupe méthyle, **caractérisé en ce qu'**on soumet à une alkylation un phénylalcanol non substitué de formule (II) 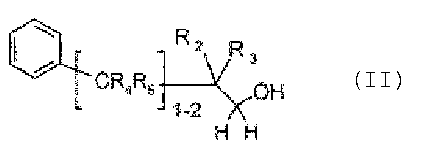 dans laquelle R₂, R₃, R₄ et R₅ ont les significations indiquées au-dessous de la formule (I), conjointement avec un halogénure d'alkyle en C₁-C₅ de formule (III)
R₁-Hal (III)
dans laquelle R₁ a la signification indiquée au-dessous de la formule (I) et Hal représente un atome d'halogène, en présence d'un catalyseur de Friedel-Crafts, pour obtenir un mélange de phénylalcanols m- et p-substitués par alkyle de formule (I), ensuite on soumet le mélange réactionnel à un traitement final, et on sépare le phénylalcanol m-substitué par alkyle de formule (I) recherché ou le phénylalcanol p-substitué par alkyle de formule (I) recherché, on renvoie dans le mélange réactionnel les autres produits formés et on les isomérise en présence d'un catalyseur de Friedel-Crafts en le phénylalcanol m- ou p-substitué par alkyle recherché.

2. Procédé selon la revendication 1 pour la préparation de phénylpropanols m- ou p-substitués de formule (IV) 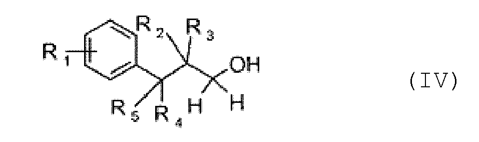 dans laquelle R₁, R₂, R₃, R₄ et R₅ ont les significations indiquées dans la revendication 1, **caractérisé en ce qu'**on soumet à une alkylation un phénylpropanol non substitué de formule (V) 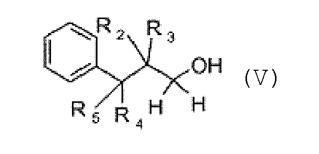 dans laquelle R₂, R₃, R₄ et R₅ ont les significations indiquées au-dessous de la formule (IV), conjointement avec un halogénure d'alkyle en C₁-C₅ de formule (III)
R₁-Hal (III)
dans laquelle R₁ et Hal ont les significations indiquées dans la revendication 1, en présence d'un catalyseur de Friedel-Crafts, pour obtenir un mélange de phénylpropanols m- et p-substitués par alkyle de formule (IV) et ensuite on soumet le mélange réactionnel à un traitement final et on sépare, de préférence sépare par distillation, le phénylpropanol m- ou p-substitué par alkyle de formule (IV) recherché, et on renvoie dans le mélange réactionnel les sous-produits formés restants et on les isomérise en présence d'un catalyseur de Friedel-Crafts en le phénylalcanol m- ou p-substitué par alkyle recherché.

3. Procédé selon la revendication 1 ou 2, **caractérisé en ce qu'**on part d'un phénylpropanol non substitué de formule (VI) 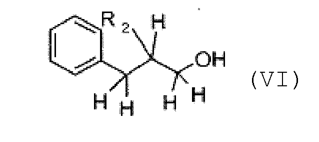 dans laquelle R₂ a la signification indiquée dans la revendication 1, on le fait réagir conjointement avec un halogénure d'alkyle en C₁-C₅ de formule (III)
R₁-Hal (III)
dans laquelle R₁ et Hal ont les significations indiquées dans la revendication 1, en présence d'un catalyseur de Friedel-Crafts, pour obtenir un phénylpropanol m- ou p-substitué par alkyle de formule (VII) 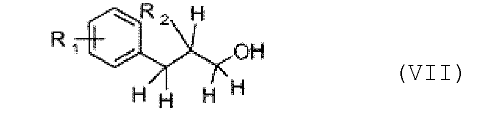 dans laquelle R₁ et R₂ ont les significations indiquées au-dessous des formules (VI) et (III) et ensuite on soumet le mélange réactionnel à un traitement final et on sépare, de préférence sépare par distillation, le phénylpropanol m- ou p-substitué par alkyle de formule (VII) recherché, et on renvoie dans le mélange réactionnel les sous-produits formés restants et on les isomérise en présence d'un catalyseur de Friedel-Crafts en le phénylpropanol m- ou p-substitué par alkyle recherché.

4. Procédé selon l'une quelconque des revendications 1 à 3, **caractérisé en ce qu'**on utilise comme halogénure d'alkyle en C₁-C₅ un halogénure d'éthyle, en particulier le bromure d'éthyle et l'iodure d'éthyle, un halogénure d'isopropyle, en particulier le chlorure d'isopropyle et le bromure d'isopropyle, un halogénure d'isobutyle, en particulier le chlorure d'isobutyle et le bromure d'isobutyle, ou un halogénure de tert-butyle, en particulier le chlorure de tert-butyle.

5. Procédé selon l'une quelconque des revendications 1 à 4, **caractérisé en ce qu'**on utilise comme catalyseur de Friedel-Crafts AlCl₃, AlBr₃, TiCl₄, ZrCl₄, VCl₃, ZnCl₂, FeBr₃, FeCl₃.

6. Procédé selon la revendication 5, **caractérisé en ce qu'**on utilise comme catalyseur de Friedel-Crafts AlCl₃ ou AlBr₃.

7. Procédé selon l'une quelconque des revendications 1 à 6, **caractérisé en ce qu'**on utilise le catalyseur de Friedel-Crafts en une quantité de 1 à 200 % en moles, en particulier comprise entre 33 et 110 % en moles, par rapport à la quantité molaire du phénylalcanol utilisé.

8. Procédé selon l'une quelconque des revendications 1 à 6, **caractérisé en ce qu'**on effectue l'alkylation à une température comprise entre 0 et 100 °C.

9. Procédé selon l'une quelconque des revendications 1 à 8, **caractérisé en ce qu'**on fait réagir du 2-méthyl-3-phénylpropanol conjointement avec un halogénure de tert-butyle, en particulier du chlorure de tert-butyle, en présence d'un catalyseur de Friedel-Crafts, en particulier en présence de trichlorure d'aluminium ou de tribromure d'aluminium, pour aboutir au 2-méthyl-3-(3- ou 4-tert-butylphényl)propanol et ensuite on soumet le mélange réactionnel à un traitement final et on sépare, de préférence sépare par distillation, le 2-méthyl-3-(3-ou 4-tert-butylphényl)propanol recherché, et on renvoie dans le mélange réactionnel les sous-produits formés restants et on les isomérise en présence d'un catalyseur de Friedel-Crafts en le 2-méthyl-3-(3- ou 4-tert-butylphényl)propanol recherché.

10. Procédé selon l'une quelconque des revendications 1 à 9, **caractérisé en ce qu'**après traitement final du mélange réactionnel on sépare le produit de réaction m-substitué recherché.

11. Procédé pour la fabrication de substances odorantes de formule (VIII) 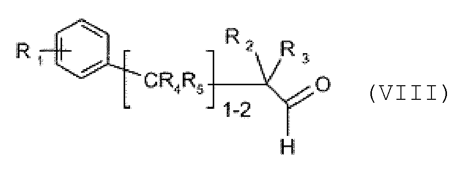 dans laquelle R₁ est lié en position m ou p au cycle phényle et représente un groupe alkyle en C₁-C₅, et R₂, R₃, R₄ et R₅ représentent, indépendamment les uns des autres, un atome d'hydrogène ou le groupe méthyle, **caractérisé en ce qu'**on soumet à une alkylation un phénylalcanol non substitué de formule (II) 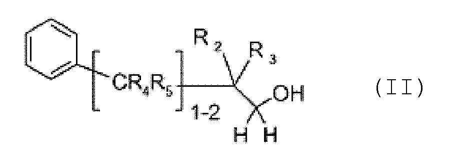 dans laquelle R₂, R₃, R₄ et R₅ ont les significations indiquées au-dessous de la formule (VIII), conjointement avec un halogénure d'alkyle en C₁-C₅ de formule (III)
R₁-Hal (III)
dans laquelle R₁ a la signification indiquée au-dessous de la formule (VIII) et Hal représente un atome d'halogène, en présence d'un catalyseur de Friedel-Crafts, pour obtenir un phénylalcanol m- ou p-substitué par alkyle de formule (I), 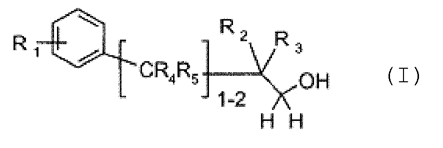 dans laquelle R₁, R₂, R₃, R₄ et R₅ ont les significations indiquées au-dessous de la formule (VIII), et ensuite on soumet le mélange réactionnel à un traitement final et on sépare, de préférence sépare par distillation, le phénylalcanol mou p-substitué par alkyle de formule (I) obtenu, et on renvoie dans le mélange réactionnel les sous-produits formés restants et on les isomérise en présence d'un catalyseur de Friedel-Crafts en le phénylalcanol m- ou p-substitué par alkyle recherché, et ensuite on le convertit par oxydation ou déshydrogénation en le phénylalcanal m- ou p-substitué par alkyle de formule (VIII).
